# EUROPÄISCHE PATENTSCHRIFT

(11) **EP 1 813 255 B1**
(45) Veröffentlichungstag und Bekanntmachung des Hinweises auf die Patenterteilung: **01.11.2017**
(21) Anmeldenummer: 07003633.0
(22) Anmeldetag: 13.04.2000
(51) Int. Cl.: A61K 8/06, A61K 8/365, A61K 8/60, A61K 31/19, A61Q 19/00, A61Q 19/10

(54) **Stabile Wirkstoffkombinationen mit einem Gehalt an grenzflächenaktiven Glucosederivaten und Hydroxycarbonsäuren**
Stable combination of active agents containing glucose derivates and hydroxycarboxylic acids
Combinaison active stable contenant des dérivés de glucose tensioactifs et des hydroxyacides

(30) Priorität: 29.04.1999 DE 19919481
(43) Veröffentlichungstag der Anmeldung: 01.08.2007
(62) Teilanmeldung aus: 00107918.5
(73) Patentinhaber: Beiersdorf AG, 20253 Hamburg (DE)
(72) Erfinder: Bormann, Angelika, 22041 Hamburg (DE); Nielsen, Jens, 24558 Henstedt-Ulzburg (DE); Herpens, Andreas, 21465 Reinbek (DE); Müller, Anja, 26789 Leer (DE)

(56) Entgegenhaltungen:
- DE-A1- 19 520 859
- US-A- 5 531 993
- US-A- 5 863 544

## Beschreibung

Die vorliegende Erfindung betrifft Wirkstoffe und Zubereitungen, solche Wirkstoffe enthaltend, welche gegen unreine Haut und Akne wirksam sind.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Übliche kosmetische Darreichungsformen sind Emulsionen, also metastabile Zwei- oder Mehrphasensysteme bei welchen die einzelnen Phasen im flüssigen Zustande vorliegen. Die gängigsten Emulsionen sind O/W- und W/O-Emulsionen. Seltenere Darreichungsformen sind multiple Emulsionen, also solche, welche in den Tröpfchen der dispergierten (oder diskontinuierlichen) Phase ihrerseits Tröpfchen einer weiteren dispergierten Phase enthalten, z.B. W/O/W-Emulsionen und O/W/O-Emulsionen.

Um die Metastabilität von Emulsionen gewährleisten zu können, sind in der Regel grenzflächenaktive Substanzen, also Emulgatoren, nötig.

Ein Nachteil insbesondere von O/W-Emulsionen ist oft deren mangelnde Stabilität gegenüber höheren Elektrolytkonzentrationen, was sich in Phasentrennung äußert. Dies kann zwar auch bei W/O-Emulsionen gelegentlich zu Problemen führen, tritt dort aber bei weitem nicht so in den Vordergrund wie bei O/W-Systemen. Zwar läßt sich diesen oft durch geeignete Wahl des Emulgatorsystems in gewissem Maße Abhilfe schaffen, es treten dann aber ebensooft andere Nachteile auf.

Es ist andererseits oft wünschenswert, bestimmte Elektrolyte einzusetzen, um deren sonstige physikalische, chemische bzw. physiologische Eigenschaften nutzen zu können. So beschreiben die DE-OS 43 29 379 und die DE-OS 44 38 588 Zubereitungen gegen unreine Haut bzw. leichte Formen von Akne, die sich durch einen Gehalt an Wollwachssäurefraktionen auszeichnen,

Bei der unreinen Haut sind neben anderen Einflüssen bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen.

Zwar sind aus der DE 197 23 733 kosmetische und dermatologische Emulsionen bekannt mit mindestens einer wäßrigen Phase, enthaltend eine wirksame Menge einer oder mehrerer grenzflächenaktiven Substanzen, gewählt aus der Gruppe der Alkylglucoside, wobei mindestens eine der wäßrigen Phasen einen oder mehrere Elektrolyte gelöst enthält, wobei die lonenstärke der wäßrigen Phasen, in der der oder die Elektrolyte gelöst vorliegen, mindestens 0,075 mol/l beträgt. Sauer eingestellte O/W-Emulsionen enthaltend Hydroxysäuren waren dem Fachmann aus US 5,863,544, US 5,531,993 und DE 195 20 859 A1 bekannt.

Diese Schriften konnten jedoch nicht den Weg zur vorliegenden Erfindung bahnen.

Eine Aufgabe der vorliegenden Erfindung war es also, Lösungswege zu kosmetischen oder dermatologischen Zubereitungen gegen Akne, insbesondere Emulsionen, bevorzugt O/W-Emulsionen, aufzudecken, welche gegenüber erhöhten Elektrolytkonzentrationen - bzw. erhöhten lonenstärken - stabil sind.

Es hat sich überraschenderweise gezeigt, und darin liegt die Lösung der Aufgabe, daß O/W-Emulsionen gegen Akne, enthaltend
(a) eine gegen unreine Haut oder Akne wirksame Menge an Milchsäure und
(b) Polyglyceryl-3-methylglucosedistearat,
   wobei
(c) die Zubereitungen auf pH-Werte von 5,5 oder kleiner abgepuffert sind den Mißständen des Standes der Technik abhilft.

Erfindungsgemäß erhältliche Emulsionen sind nicht nur erstaunlich stabil, sie sind auch besser gegen unreine Haut und Akne wirksam als der Stand der Technik hätte erwarten lassen. Darüberhinaus zeichnen sie sich auch durch gesteigerte Hautverträglichkeit und bessere kosmetische Erscheinung aus.

Milchsäure (2-Hydroxypropansäure) ist durch folgende chemische Struktur gekennzeichnet : 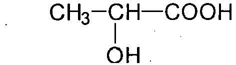 Als "Polyglyceryl(3)-Methylglucosedistearat" (PGMS) bezeichnete Emulgatorkombinationen sind unter der Warenbezeichnung Tego Care® erhältlich.

Erfindungsgemäß können als günstige, aber dennoch fakultativ zu verwendende Antioxidantien alle für kosmetische und/oder dermatologische Anwendungen geeigneten oder gebräuchlichen Antioxidantien verwendet werden.

Es ist auch von Vorteil, den erfindungsgemäßen Zubereitungen Antioxidantien zuzusetzen. Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-,γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsutfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung. Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Antioxidantien, Insektenrepellentien, Alkohol, Wasser, Salze, antimikrobiell, proteolytisch oder keratolytisch wirksame Substanzen usw.

Den erfindungsgemäßen Zubereitungen sind Puffersubstanzen zugefügt. Die Zubereitungen sind auf pH-Werte von 5,5 oder kleiner abgepuffert. Entsprechend können die erfindungsgemäßen Zusammensetzungen, je nach ihrem Aufbau, beispielsweise verwendet werden als Hautschutzcrème, Reinigungsmilch, Sonnenschutzlotion, Nährcreme, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Zur Anwendung werden die erfindungsgemäßen kosmetischen und dermatologischen Zubereitungen in der für Kosmetika üblichen Weise auf die Haut in ausreichender Menge aufgebracht.

Es ist auch möglich und vorteilhaft im Sinne der vorliegenden Erfindung, die erfindungsgemäßen Wirkstoffkombinationen in wäßrige Systeme bzw. Tensidzubereitungen zur Reinigung der Haut einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösemittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein. Erfindungsgemäße Emulsionen, z.B. in Form einer Hautschutzcreme, einer Hautlotion, einer kosmetischen Milch, beispielsweise in Form einer Sonnenschutzcreme oder einer Sonnenschutzmilch, enthalten z.B. die genannten Fette, Öle, Wachse und anderen Fettkörper, sowie Wasser und einen Emulgator, wie er üblicherweise für einen solchen Typ der Formulierung verwendet wird.

Zubereitungen können auch günstig als Gele vorliegen, die neben den erfindungsgemäßen Wirkstoffkombinationen und dafür üblicherweise verwendeten Lösemitteln, üblicherweise Alkohole niedriger C-Zahl, z.B. Ethanol, Isopropanol, 1,2-Propandiol, Glycerin und Wasser bzw. ein vorstehend genanntes Öl, dann noch organische Verdickungsmittel enthalten, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Siliciumdioxid und/oder Aluminiumsilikate wie beispielsweise Bentonite, oder ein Gemisch aus Polyethylenglykol und Polyethylenglykolstearat oder -distearat. Das oder die Verdickungsmittel sind im Gel z.B. in einer Menge zwischen 0,1 und 30 Gew.-%, bevorzugt zwischen 0,5 und 15 Gew.-%, enthalten. Erfindungsgemäße feste Stifte enthalten z.B. natürliche oder synthetische Wachse, Fettalkohole oder Fettsäureester.

Als Treibmittel für erfindungsgemäße, aus Aerosolbehältern versprühbare kosmetische und/oder dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Die erfindungsgemäßen kosmetischen oder dermatologischen Zubereitungen enthalten bevorzugt

| | |
|---|---|
| 0,001 - 0,20 Gew.-% | Retinol und oder dessen Ester |
| 0,001 - 5,00 Gew.-% | einer oder mehreren α-Hydroxycarbonsäuren und/oder α-Ketocarbonsäuren, insbesondere Citronensäure, und |
| 0,001 - 10,00 Gew.-% | eines oder mehrerer Stoffe aus der Gruppe der Monoglycerinmonocarbonsäure-monoester, der Diglycerin-monocarbonsäure-monoester sowie der Triglycerin-monocarbonsäure-monoester |

jeweils bezogen auf das Gesamtgewicht der Zubereitungen.

Bevorzugt können sie außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische und/oder dermatologische Zubereitungen zur Verfügung zu stellen, die die Haut vor dem gesamten Bereich der ultravioletten Strahlung schützen.

Enthalten die erfindungsgemäßen Emulsionen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, z.B. 3-(4-Methylbenzyliden)campher, 3-Benzylidencampher;
- 4-Aminobenzoësäure-Derivate, z.B. 4-(Dimethylamino)-benzoësäure(2-ethylhexyl)ester, 4-(Dimethylamino)benzoësäureamylester;
- Ester der Zimtsäure, z.B. 4-Methoxyzimtsäure(2-ethylhexyl)ester, 4-Methoxyzimtsäureisopentylester;
- Ester der Salicylsäure, z.B. Salicylsäure(2-ethylhexyl)ester, Salicylsäure(4-isopropylbenzyl)ester, Salicylsäurehomomenthylester,
- Derivate des Benzophenons, z.B. 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, z.B. 4-Methoxybenzalmalonsäuredi(2-ethylhexyl)ester, - 2,4,6-Trianilino-(p-carbo-2'-ethyl-1'-hexyloxy) -1,3,5-triazin.
Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, wie z.B. 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Enthalten die erfindungsgemäßen Emulsionen UVA-Filtersubstanzen, können diese erfindungsgemäß vorteilhaft gewählt werden aus der Gruppe der Derivate des Dibenzoylmethans, z.B. 1-(4'-tert.Butylphenyl)-3-(4'-methoxyphenyl)propan-1,3-dion und 1-Phenyl-3-(4'-isopropylphenyl)propan-1,3-dion.

Die erfindungsgemäßen Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen bzw. Abwandlungen davon. Besonders bevorzugt handelt es sich um Pigmente auf der Basis von Titandioxid.

Bei erfindungsgemäßen kosmetischen und dermatologischen Zubereitungen für die Verwendung an behaarter Haut handelt es sich beispielsweise um Shampoonierungsmittel, Zubereitungen, die beim Spülen der Haare vor_oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen und/oder dermatologischen Zubereitungen enthalten gegebenenfalls zusätzliche Wirkstoffe, Hilfs- und/oder Zusatzstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektrolyte sowie zusätzliche Substanzen zum Rückfetten der Haare bzw. der Kopfhaut.

Erfindungsgemäße wäßrige kosmetische Reinigungsmittel mit einem Gehalt an den erfindungsgemäßen Wirkstoffkombinationen bzw. für die wäßrige Reinigung bestimmte wasserarme oder wasserfreie Reinigungsmittelkonzentrate können anionische, nichtionische und/oder amphotere Tenside enthalten, beispielsweise
- herkömmliche Seifen, z.B. Fettsäuresalze des Natriums
- Alkylsulfate, Alkylethersulfate, Alkan- und Alkylbenzolsulfonate
- Sulfoacetate
- Sulfobetaine
- Sarcosinate
- Amidosulfobetaine
- Sulfosuccinate
- Sulfobernsteinsäurehalbester
- Alkylethercarboxylate
- Eiweiß-Fettsäure-Kondensate
- Alkylbetaine und Amidobetaine
- Fettsäurealkanolamide
- Polyglycolether-Derivate

Kosmetische Zubereitungen, die kosmetische Reinigungszubereitungen für die Haut darstellen, können in flüssiger oder fester Form vorliegen. Sie enthalten vorzugsweise mindestens eine anionische, nicht-ionische oder amphotere oberflächenaktive Substanz oder Gemische daraus, mindestens einen erfindungsgemäßes Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen.

Kosmetische Zubereitungen, die ein Shampoonierungsmittel darstellen, enthalten neben einem wirksamen Gehalt an den erfindungsgemäßen Wirkstoffkombinationen vorzugsweise mindestens eine anionische, nicht-ionische oder amphotere oberflächenaktive Substanz oder Gemische daraus, gegebenenfalls einen erfindungsgemäßes Elektrolyten und Hilfsmittel, wie sie üblicherweise dafür verwendet werden. Die oberflächenaktive Substanz kann in einer Konzentration zwischen 1 Gew.-% und 94 Gew.-% in dem Shampoonierungsmittel vorliegen.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wir, so handelt es sich dabei z.B. um wäßrige oder wäßrig-alkoholische Lösungen, die gegebenenfalls oberflächenaktive Substanzen enthalten, bevorzugt nicht-ionische oder kationische oberflächenaktive Substanzen, deren Konzentration zwischen 0,1 und 10 Gew.-%, vorzugsweise zwischen 0,2 und 5 Gew.-%, liegen kann. Diese kosmetischen und/oder dermatologischen Zubereitungen können auch Aerosole mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie die erfindungsgemäßen Wirkstoffkombinationen. Die Menge der verwendeten Polymeren liegt z.B. zwischen 0,1 und 10 Gew.-%, bevorzugt zwischen 0,1 und 3 Gew.-%.

Erfindungsgemäße kosmetische Zubereitungen zur Behandlung und Pflege der behaarten Haut, die erfindungsgemäße Wirkstoffkombinationen enthalten, können als Emulsionen vorliegen, die vom nicht-ionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Erfindungsgemäße Emulsionen zur Nagelpflege enthalten z.B. die genannten Fette, Öle, Wachse und andere Fettkörper, sowie Wasser und einen Emulgator, wie er üblicherweise für einen solchen Typ der Formulierung verwendet wird.

Gele zur Pflege und/oder Wiederherstellung der Nägel enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdikkungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Ansonsten gelten für diese Gruppe an kosmetischen und/oder dermatologischen Zubereitungen die üblichen Anforderungen, die der Fachmann an solche Zubereitungen und deren Inhaltsstoffe stellt.

Die folgenden Beispiele wollen die vorliegende Erfindung erläutern, ohne daß eine Beschränkung auf den Gehalt der Beispiele beabsichtigt ist. Die Mengenangaben bedeuten stets, sofern nichts Anderes angegeben ist, Gewichts-%, bezogen auf das Gesamtgewicht der jeweiligen Zubereitungen.

**Beispiel 1**

| | % |
|---|---|
| Polyglyceryl-3-methylglucosedistearat | 6,000 |
| Glycerin | 5,000 |
| Sorbitanstearat | 3,000 |
| Cetylstearylalkohol | 2,000 |
| Milchsäure | 2,000 |
| PEG-150-Distearat | 0,500 |
| Xanthangummi | 0,500 |
| Natriumhydroxid | 0,450 |
| Titandioxid | 0,350 |
| Diazolidinylharnstoff | 0,250 |
| Wasser | ad 100,00 |

**Beispiel 2**

| | % |
|---|---|
| Titandioxid | 10,000 |
| Cyclomethicon | 10,000 |
| Glycerin | 10,000 |
| Polyglyceryl-3-methylglucosedistearat | 4,000 |
| Sorbitanstearat | 1,000 |
| Cetylstearylalkohol | 6,000 |
| Milchsäure | 3,000 |
| Eisenoxide | 2,000 |
| PEG-150-Distearat | 2,000 |
| Xanthangummi | 0,500 |
| Natriumhydroxid | 0,450 |
| Diazolidinylharnstoff | 0,250 |
| Bisabolol | 0,100 |
| Konservierungsmittel, Farbstoffe, Parfum | q.s. |
| Wasser | ad 100,00 |

**Beispiel 3**

| | % |
|---|---|
| Polyglyceryl-3-methylglucosedistearat | 6,000 |
| Glycerin | 5,000 |
| Sorbitantetrastearat | 3,000 |
| Cetylstearylalkohol | 1,000 |
| Milchsäure | 1,000 |
| PEG-150-Distearat | 0,500 |
| Xanthangummi | 0,500 |
| Natriumhydroxid | 0,450 |
| Titanium Dioxide | 0,350 |
| Diazolidinylharnstoff | 0,250 |
| Konservierungsmittel, Farbstoffe, Parfum | q.s. |
| Wasser | ad 100,00 |

**Beispiel 4**

| | % |
|---|---|
| Polyglyceryl-3-methylglucosedistearat | 5,500 |
| Glycerin | 15,000 |
| Sorbitanstearat | 3,500 |
| Cetylstearylalkohol | 3,000 |
| Milchsäure | 1,500 |
| PEG-150-Distearat | 1,500 |
| Natriumhydroxid | 0,450 |
| Diazolidinylharnstoff | 0,250 |
| Konservierungsmittel, Farbstoffe, Parfum | q.s. |
| Wasser | ad 100,00 |

**Beispiel 5**

| | % |
|---|---|
| Polyglyceryl-3-methylglucosedistearat | 6,000 |
| Glycerin | 5,000 |
| Sorbitantristearat | 3,000 |
| Cetylstearylalkohol | 2,000 |
| Milchsäure | 2,000 |
| Xanthangummi | 0,500 |
| Natriumhydroxid | 0,450 |
| Titandioxid | 0,350 |
| Konservierungsmittel, Farbstoffe, Parfum | q.s. |
| Wasser | ad 100,00 |

**Beispiel 6**

| | % |
|---|---|
| Polyglyceryl-3-methylglucosedistearat | 6,000 |
| Glycerin | 5,000 |
| Sorbitanstearat | 3,000 |
| Stearylalcohol | 2,000 |
| Milchsäure | 2,000 |
| PEG-150-distearat | 2,500 |
| Xanthangummi | 1,500 |
| Natriumhydroxid | 0,450 |
| Titandioxid | 0,350 |
| Diazolidinylharnstoff | 0,250 |
| Konservierungsmittel, Farbstoffe, Parfum | q.s. |
| Wasser | ad 100,00 |

## Patentansprüche

1. O/W-Emulsionen gegen Akne, enthaltend
(a) eine gegen unreine Haut oder Akne wirksame Menge an Milchsäure und
(b) Polyglyceryl-3-methylglucosedistearat, wobei
(c) die Zubereitungen auf pH-Werte von 5,5 oder kleiner abgepuffert sind.

2. O/W-Emulsionen nach einem der vorstehenden Ansprüche, **dadurch gekennzeichnet, dass** die Gesamtmenge an Polyglyceryl-3-methylglucosedistearat in den fertigen kosmetischen oder dermatologischen Zubereitungen aus dem Bereich von 0,1 - 15,0 Gew.-% gewählt wird, bezogen auf das Gesamtgewicht der Zubereitungen.

## Claims

1. O/W emulsions to counter acne, comprising
(a) an amount of lactic acid effective against blemished skin or acne and
(b) polyglyceryl-3 methylglucose distearate, wherein
(c) the preparations are buffered to pH values of 5.5 or less.

2. O/W emulsions according to one of the preceding claims, **characterized in that** the total amount of polyglyceryl-3 methylglucose distearate in the finished cosmetic or dermatological preparations is selected from the range of 0.1 - 15.0% by weight, based on the total weight of the preparations.

## Revendications

1. Émulsions H/E contre l'acné, contenant
(a) une quantité d'acide lactique active contre une peau impure ou l'acné et
(b) du Polyglycéryl-3 Méthylglucose Distéarate,
(c) les préparations étant tamponnées à des valeurs de pH de 5,5 ou moins.

2. Émulsions H/E selon l'une quelconque des revendications précédentes, **caractérisées en ce que** la quantité totale de Polyglycéryl-3 Méthylglucose Distéarate dans les préparations cosmétiques ou dermatologiques finies est choisie dans la plage de 0,1 - 15,0% en poids, par rapport au poids total des préparations.
